# EUROPÄISCHE PATENTSCHRIFT

(11) **EP 1 933 276 B1**
(45) Veröffentlichungstag und Bekanntmachung des Hinweises auf die Patenterteilung: **30.06.2010**
(21) Anmeldenummer: 06025561.9
(22) Anmeldetag: 11.12.2006
(51) Int. Cl.: A61B 17/00, G06T 7/00, A61B 19/00

(54) **Mehrbandtracking- und Kalibrier-System**
Multiband tracking and calibration system
Système multibande d'étalonnage et de localisation

(43) Veröffentlichungstag der Anmeldung: 18.06.2008
(73) Patentinhaber: BrainLAB AG, 85622 Feldkirchen (DE)
(72) Erfinder: Feilkas, Thomas, 85567 Grafing (DE); Weiser, Manfred, Dr., 81673 München (DE)
(74) Vertreter: Schwabe - Sandmair - Marx

(56) Entgegenhaltungen:
- EP-A- 1 667 067
- EP-A2- 1 138 256
- WO-A-01/01854
- WO-A-96/41481
- US-A1- 2003 135 092
- US-A1- 2003 208 125
- US-A1- 2005 285 038

## Beschreibung

Die vorliegende Erfindung bezieht sich auf ein Mehrbandtracking- und/oder Kalibrier- oder Registrier-System, um ein zum Beispiel medizinisch verwendbares Instrument, wie zum Beispiel eine Schere oder einen Spreader, oder ein Implantat zu kalibrieren oder einen Patienten oder eine zum Beispiel anatomische Struktur zu registrieren und insbesondere um die geometrische Struktur oder Konfiguration eines Instrumentes oder Implantates zu erfassen.

Um ein medizinisches Instrument in der bildgeführten Chirurgie einsetzen zu können, muss dieses Instrument kalibriert bzw. verifiziert oder validiert sein, d,h, es muss bekannt sein, wie die genaue Abmessung, Konfiguration oder Anordnung des Instrumentes ist, da ansonsten aufgrund zum Beispiel einer Verbiegung einer Instrumentenspitze in ein gesundes Gewebe eingegriffen wird, welches neben dem eigentlich zu behandelnden Gewebe liegt, in welches eine unverbogene Instrumentenspitze eingreifen würde.

Eine Vorrichtung und ein Verfahren zum Kalibrieren eines gebogenen Elements sind aus der EP 1 413 258 A1 bekannt, wobei das gebogene Element mit einem Navigationselement verbunden ist, an eine Kalibrier-Vorrichtung angelegt wird und in Anlage an dieser Kalibrier-Vorrichtung bewegt wird, bis das Element kalibriert ist.

Aus der EP 1 369 090 A1 ist die Navigations-Kalibrierung medizinischer Instrumente oder Implantate bekannt, wobei eine räumliche Position des Instruments oder Implantats mittels eines medizinischen Navigationssystems ermittelt wird, um die Relativposition des Instruments oder Implantats gegenüber anatomischen Daten zu ermitteln, wobei die räumliche Ausrichtung eines mehrdimensional ausgestalteten, funktionellen Abschnitts des Instruments oder Implantats ermittelt wird.

Die US 6,428,547 B1 beschreibt das Erkennen der Form eines Behandlungsgerätes, wobei das Behandlungsgerät in einem computergesteuerten kameraunterstützten Navigationssystem mittels eines Marker-Arrays, welches an dem Behandlungsgerät angebracht ist, referenziert wird, wobei Projektionen des Behandlungsgerätes mittels Röntgenaufnahmen detektiert werden und die Form des Behandlungsgerätes diesem in dem Navigationssystem über die Position des Marker-Arrays in den Projektionen zugeordnet wird.

Aus der US 6,724,922 B1 ist ein Verifikationsverfahren für Positionen in Kamerabildern bekannt.

Die DE 199 44 516 A1 beschreibt ein Verfahren zur Erfassung einer Objektform, wobei ein Kamerabild des Objektes erstellt wird, ein Umriss des Objektes in einer ersten Ebene durch eine mit der Kamera verbundene Auswertungseinheit erfasst wird, der Fokussierungsabstand der Kamera verändert wird, ein Umriss des Objektes in einer zweiten Ebene durch die Auswertungseinheit erfasst wird und diese Schritte wiederholt werden, bis eine ausreichende Zahl von Umrissen erfasst ist, so dass die räumliche Form des Objektes festgestellt werden kann.

Aus der EP 1 667 067 A1 der Anmelderin ist ein Verfahren zum Kalibrieren eines Instruments oder Implantats bekannt, an welchem mindestens ein Marker angebracht ist, wobei die Position des Instrumentes oder Implantats im Raum mittels des mindestens einen Markers ermittelt wird, die Umrisse oder Ansicht oder Geometrie des Instrumentes oder Implantats von mindestens einer Seite optisch erfasst wird und mit korrespondierenden Umrissen, Ansichten oder Geometrien von gespeicherten Präkalibrier-Daten des Instrumentes oder Implantats zur Darstellung eines Instrumenten-Modells verglichen wird, um zu ermitteln, ob das Instrument oder Implantat kalibriert ist, sowie eine Vorrichtung zum Kalibrieren eines Instrumentes oder Implantats mit einer Recheneinheit, welche mit einem Speicher verbunden ist, in dem Präkalibrier-Daten des Instrumentes abgelegt sind, mindestens einer Kamera, mit welcher an dem Instrument angebrachte Marker erfasst werden können und mindestens einer Video-Kamera, wobei die mindestens eine Kamera und die Videokamera mit der Recheneinheit verbunden sind und ein definiertes Lageverhältnis zueinander aufweisen, das von der Recheneinheit bestimmt werden kann, um die von der Videokamera erfassten Bilddaten des Instruments unter Verwendung der von der Kamera erfassten Positionsinformation bezüglich des Instrumentes so auszuwerten, dass diese mit den Präkalibrier-Daten verglichen werden können.

Aus der US 2005/0285038 A1 ist eine Bilderfassungsvorrichtung bekannt, wobei ein Bilderfassungssystem sichtbares Licht und Licht eines anderen Spektrums erfassen kann. Bei einer Ausführungsform ist ein Strahlteiler vorgesehen, um ein von einer Linse kommendes Bild auf mehrere CCD-Elemente aufzuteilen.

Ein System zum Bestimmen der räumlichen Position eines Körpers ist aus der WO 99/17133 und der WO 99/30182 bekannt.

Die US 2004/0002642 A1 beschreibt ein Mess-System zur Verfolgung einer Pose eines in einem Bezugs-Koordinatensystem, wie zum Beispiel ein dreidimensionales Koordinatensystem, darstellbaren Objekts. Das System umfasst einen vor-definierten physikalischen Marker zum starren Koppeln an das Objekt.

Es ist eine Aufgabe der vorliegenden Erfmdung ein Verfahren und eine Vorrichtung zum einfachen Tracken, Kalibrieren oder Registrieren eines Körpers, wie zum Beispiel eines bevorzugt medizinisch verwendbaren Instrumentes oder Implantats vorzuschlagen.

Diese Aufgabe wird durch die Gegenstände der unabhängigen Ansprüche gelöst. Vorteilhafte Ausführungsformen ergeben sich aus den abhängigen Ansprüchen.

Das erfindungsgemäße Kamerasystem weist mindestens zwei optische Erfassungssysteme oder Kameras auf, welche in einem vorgegebenen Abstand von zum Beispiel 10 bis 1000 mm beispielsweise innerhalb eines Gehäuses angeordnet sind. Erfindungsgemäß ist mindestens ein und vorteilhaft jedes optische Erfassungssystem so ausgelegt, dass gleichzeitig oder hintereinander Licht in einem unterschiedlichen Spektral- oder Wellenbereich erfasst werden kann, wie zum Beispiel Infrarot, sichtbares Licht (zum Beispiel im Bereich von etwa 380 bis etwa 780 nm) und Licht mit einer Wellenlänge im ultravioletten Bereich. Hierdurch ist es möglich, dass mit dem erfindungsgemäßen Kamerasystem sowohl zum Beispiel in Verbindung mit einem an sich bekannten Navigationssystem ein Tracking-Verfahren durchgeführt werden kann, wobei die Kameras oder Erfassungssysteme zum Beispiel Licht im Infrarotbereich erfassen, während andererseits mit den beiden Erfassungssystemen oder Kameras auch zum Beispiel Licht im sichtbaren Bereich erfasst werden kann, um beispielsweise ein Instrument zu kalibrieren, verifizieren oder validieren. Durch das Integrieren mindestens zweier Erfassungsvorrichtungen zur Erfassung von zum Beispiel sichtbarem Licht in die Kameras zur Erfassung von zum Beispiel IR-Licht eines bekannten Stereoskopie-Kamerasystems kann ohne eine signifikante Erhöhung des Gewichts des gesamten Kamerasystems und auch ohne Set-up- oder Synchronisations-Probleme auf einfache Weise eine Vorrichtung geschaffen werden, welche nicht nur zum Tracken von zum Beispiel IR-Licht reflektierenden Markern verwendet werden kann, sondern welche auch das Auswerten von optischen Informationen zum Beispiel im sichtbaren Wellenlängenbereich ermöglicht. Somit kann das erfindungsgemäße Kamerasystem auch an Stelle eines bekannten zum Beispiel nur IR-Licht detektierenden Kamerasystems eingesetzt werden, wobei diese Funktionalität beibehalten und um die optische Erfassung in einem anderen Wellenlängenbereich erweitert wird, um zum Beispiel mittels Stereoskopieaufnahmen eine Kalibrierung vorzunehmen.

Sofern von Wellenlängen- oder Spektralbereichen gesprochen wird, soll erfindungsgemäß auch Licht einer zum Beispiel konstanten Wellenlänge erfasst sein.

Die Modifikation einer Kamera zur Erfassung von Licht eines weiteren anderen Wellenlängenbereiches kann zum Beispiel durch das Vorsehen eines Strahlteilers, wie zum Beispiel eines Prismas oder eines halbdurchlässigen Spiegels, erfolgen, welcher in dem Strahlengang des zum Beispiel durch eine Linse oder Optik eintretenden Lichtes liegt. Das durch den Strahlteiler aufgeteilte eintretende Licht kann zum Beispiel nach der Strahlteilung auf ein Erfassungselement, wie zum Beispiel einen ersten Sensor, zum Beispiel ein bekanntes CCD-Array zur Erfassung von Licht eines ersten Wellenlängenbereiches, und auf einen zweiten Sensor zur Erfassung von Licht eines zweiten anderen Wellenlängenbereiches treffen. Ein System zur Auftrennung von eintretendem Licht ist zum Beispiel in der WO 96/41481 und insbesondere in dem in deren Figur 7 gezeigten Ausführungsbeispiel beschrieben, wobei die technische Lehre der WO 96/41481 bezüglich der Aufteilung von Licht in verschiedene Wellenlängen oder -bereiche und der getrennten Erfassung einzelner Spektralbereiche eines eintretenden Lichtes in die vorliegende Anmeldung aufgenommen werden.

Unter dem Begriff Erfassungselement oder CCD-Element soll im Sinne der Erfindung jedes Erfassungselement verstanden werden, mit welchem ein optisches Signal erfasst und zum Beispiel in ein elektrisches Signal umgewandelt werden kann, wie zum Beispiel eine separate Kamera, ein CCD-Chip, ein CMOS Sensor oder ähnliches.

Es wird angemerkt, dass auch mehr als zwei Erfassungselemente oder CCD-Elemente in einer einzelnen Kamera vorgesehen sein können, um mehr als zwei Spektralbereiche zu erfassen. Dabei können beispielsweise zusätzliche halbdurchlässige Spiegel und/oder Prismen und optional auch zusätzliche Filterelemente in den Kameras verwendet werden, um zum Beispiel UV-Licht, sichtbares Licht und Infrarot-Licht gleichzeitig oder sequentiell zu erfassen.

Weiterhin ist es möglich, dass ein oder mehrere optische Filter verwendet werden, welche vor ein oder mehrere Erfassungselemente, zum Beispiel vor ein CCD-Array, oder in dem Strahlengang des zu dem jeweiligen Erfassungselement gelangenden Lichtes platziert werden, um so die Erfassung von Licht mit unterschiedlicher Wellenlänge durch verschiedene Sensoren gleicher oder unterschiedlicher Bauart zu ermöglichen. Ebenso ist es möglich, dass jeweils speziell zur Erfassung eines bestimmten Wellenlängeukreiches ausgebildete Sensoren verwendet werden. Ist zum Beispiel pro Kamera des erfindungsgemäßen Kamerasystems nur ein einzelnes Erfassungselement, wie zum Beispiel nur ein CCD-Array, vorgesehen, so kann die Erfassung von Licht unterschiedlicher Wellenlängenbereiche dadurch ermöglicht werden, dass zunächst zum Beispiel ein optischer Filter, welcher nur Licht eines ersten Wellenlängenbereichs durchlässt, vor dem Erfassungselement oder in dem Strahlengang des auf das Erfassungselement treffenden Lichtes platziert wird, so dass nur Licht des ersten Wellenlängenbereiches von dem Erfassungselement erfasst werden kann. Nach einer vorgegebenen Zeit, in welcher das Licht des ersten Wellenlängenbereiches erfasst wurde, kann der Filter herausgenommen oder zum Beispiel durch einen anderen Filter ersetzt werden, welcher an der gleichen oder zum Beispiel auch an einer anderen Stelle in den Strahlengang des Lichtes gebracht wird, um Licht eines zweiten Wellenlängenbereiches zu erfassen.

Ein erfindungsgemäß verwendbarer Filter kann sowohl ein passives Filterelement sein, welches sich nicht verändert wie zum Beispiel ein Farbfilter, oder ein aktiv steuerbarer Filter, wie zum Beispiel ein Polarisationsfilter.

Weiterhin ist es möglich ein erfindungsgemäßes Kamerasystem zum Beispiel durch das Vorsehen eines beweglichen Spiegels zu schaffen, welcher zum Beispiel in einer ersten Position Licht auf ein erstes Erfassungselement und in einer zweiten zum Beispiel weggeklappten oder gedrehten Position Licht auf ein zweites Erfassungselement lenkt, Dabei kann der bewegliche Spiegel zum Beispiel wie in einer an sich bekannten Spiegelreflexkamera aufgebaut sein und durch eine wie zum Beispiel aus einer Spiegelreflexkamera bekannte Mechanik bewegt werden, um je nach gewünschtem zu erfassenden Wellenlängenbereich das auf den Spiegel auftreffende Licht zu dem jeweils geeigneten Sensor zu lenken.

Die Bildwechselfrequenz, mit welcher eine Kamera beispielsweise nur sequentiell Licht unterschiedlicher Wellenlänge erfasst, kann beispielsweise im Bereich von 50 ms pro Bild liegen, so dass zum Beispiel in einer Sekunde jeweils 10 Bilder in einem ersten Wellenlängenbereich und jeweils 10 Bilder in einem zweiten davon verschiedenen Wellenlängenbereich erfasst werden.

Da bei allen oben beschriebenen Ausführungsformen des erfindungsgemäßen Kamerasystems die gleiche Optik, durch welche Licht in die Kamera eintritt, verwendet werden kann, können auf einfache Weise die korrespondierenden Pixel, also ein Pixel-zu-Pixel Verhältnis, zwischen den zwei Abbildungs-Modalitäten bestimmt werden. Beispielsweise kann die Transformationsvorschrift zur Abbildung eines Pixels in einem ersten zum Beispiel IR-Bild auf ein korrespondierendes Bild zum Beispiel im sichtbaren Bereich bekannt sein, so dass bestimmt werden kann, wo die im IR-Bereich gut sichtbaren Marker an dem im sichtbaren Licht gut erkennbaren medizinischen Instrument angeordnet sind, um so das Instrument tracken und gleichzeitig auch kalibrieren zu können. Idealerweise kann zum Beispiel eine direkte Abbildung (mapping) der jeweiligen Pixel eines in einem ersten Wellenlängenbereich aufgenommenen Bildes auf das korrespondierende Pixel eines in einem zweiten anderen Wellenlängenbereich aufgenommenen Bildes vorliegen, so dass die von dem gleichen oder zwei unterschiedlichen Erfassungselementen erfassten Bilder in zwei unterschiedlichen Wellenlängenbereichen zum Beispiel einfach übereinander gelegt werden können, um die kombinierte Information aus der Erfassung beider Wellenlängenbereiche in einem einzigen Bild zur Verfügung zu haben.

Im Gegensatz zur Verwendung von externen Bildquellen muss bei dem erfindungsgemäßen Kamerasystem kein Synchronisations- Verfahren durchgeführt werden, da die Synchronisation der einzelnen Kamera-Baugruppen oder Kamera-Teilelemente, wie zum Beispiel beweglicher Spiegel oder Filter oder verschiedener Erfassungselemente, bereits bei oder nach der Herstellung des Kamerasystems durch Hardware-, Firmware- oder Software-Einstellung oder Anpassung gelöst werden kann, so dass zum Beispiel bei dem Einsatz des Kamerasystems zur Navigation korrespondierende Stereo-Videobilder voll synchronisiert zusätzlich zum Tracken der IR-Marker zur Verfügung gestellt werden können. Hierzu muss das Kamerasystem lediglich kalibriert werden, was beispielsweise vor der ersten Anwendung zum Beispiel durch das Erfassen zweier Testbilder und den nachfolgenden Abgleich der beiden Testbilder, zum Beispiel zum Bestimmen der oben erwähnten Transformationsvorschrift, durchgeführt werden kann.

Mit dem erfindungsgemäßen Kamerasystem kann somit zum Beispiel eine berührungslose Verifikation eines Instrumentes durchgeführt werden, wobei im Vergleich zu bekannten Verfahren auf die Verwendung eines Kalibrier-Instrumentes, wie zum Beispiel einer Instrumenten-Kalibrier-Matrix (Instrument Calibration Matrix; ICM), verzichtet werden kann.

Aus den mindestens zwei Aufnahmen eines Objektes kann auch ein Modell der dreidimensionalen Oberfläche des Objektes rekonstruiert werden, wie zum Beispiel in dem Artikel "Shape from Stereo Using Fine Correlation: Method and Error Analysis" von Frédéric Devernay und Olivier Faugeras beschrieben, welcher bezüglich der technischen Lehre der Rekonstruktion oder Erzeugung einer dreidimensionalen Oberfläche oder eines dreidimensionalen Oberflächenmodells aus zum Beispiel einer Stereo-Aufnahme in die vorliegende Erfindung aufgenommen wird.

Vorteilhaft können eine oder mehrere Lichtquellen, wie zum Beispiel Leuchtdioden, Laser oder Lampen, an einer oder mehreren Kameras, zum Beispiel um eine Lichteintrittsöffnung oder Linse der Kameras herum, angeordnet werden. Vorteilhaft sind die Lichtquellen an der jeweiligen Kamera so ausgelegt, dass Licht in mindestens einem der von dem Kamerasystem erfassbaren Wellenlängenbereiche emittiert wird, wie zum Beispiel Infrarot-Licht, welches zum Beispiel an reflektierenden Oberflächen, beispielsweise einem Marker, reflektiert und wieder zur Kamera zurückgesandt wird. Es ist auch möglich, dass Lichtquellen unterschiedlicher Art an einer Kamera vorgesehen sind, wie zum Beispiel Lampen zur Emission von sichtbarem Licht, LED's oder Laser. Wird als ein Licht aussendendes oder Projektions-Element zum Beispiel ein Laser oder ein Video-Projektor verwendet, so kann mit so genanntem "strukturiertem Licht" ("structured light") die Rekonstruktion von dreidimensionalen Oberflächen vereinfacht werden, wie in dem Artikel "3-D Computer Vision Using Structured Light: Design, Calibration, and Implementation issues" von Fred W. DePiero and Mohan M. Trivedi, in Advances in Computers Vol. 43, Seiten 243 bis 278, depiero96computer, 1996 beschrieben.

Eine vor den jeweiligen Erfassungselementen der Kamera liegende Optik kann als variable Optik vorgesehen sein, um Einstellvorgänge, wie zum Beispiel Zoom oder Fokus, welche auch aus der Fotografie bekannt sind, vornehmen zu können. Dabei kann die variable Optik beispielsweise manuell oder elektronisch eingestellt werden, um das Kamerasystem zum Beispiel für die möglichst optimale Erfassung eines Objektes in einem vorgegebenen Abstand von dem Kamerasystem zu konfigurieren.

Vorzugsweise ist eine Recheneinheit mit beiden Kameras verbunden, welche die von den jeweiligen Kameras aufgenommenen Bilder enthalten und zum Beispiel als Stereobilder verarbeiten oder auswerten kann, um beispielsweise nach Durchführung eines 3D-Rekonstruktionsverfahrens eine Kalibrierung vorzunehmen.

Optional können bei dem erfindungs gemäßen Kamerasystem auch drei oder mehr Kameras verwendet werden, welche vorzugsweise jeweils so ausgelegt sind, dass Licht in mindestens zwei verschiedenen Wellenbereichen erfasst werden kann.

Gemäß einem weiteren Aspekt bezieht sich die Erfindung auf ein Navigationssystem mit einem wie oben beschriebenen Kamerasystem.

Die Erfindung bezieht sich weiterhin auf eine Vorrichtung zum Kalibrieren eines bevorzugt medizinisch verwendbaren Instrumentes, an welchem mindestens ein Marker oder ein Referenzstern angebracht ist, mit einer Recheneinheit und einem damit verbundenen Speicher, in welchem Präkalibrier-Daten mindestens eines medizinischen Instrumentes abgelegt sind. Weiterhin ist mindestens eine wie oben beschriebene Kamera vorgesehen, mit welcher sowohl die an dem Instrument angebrachten Marker zum Beispiel mittels reflektiertem IR-Licht erfasst werden können, wobei die Kamera mit einer Recheneinheit verbunden ist, welche die räumliche Position des Instrumentes anhand der erfassten Marker-Bilddaten und optional anhand der Präkalibrierdaten ermitteln kann. Die Vorrichtung weist weiterhin eine Kamera auf, welche in die obige Kamera integriert ist und Licht eines weiteren Wellenlängenbereichs erfassen kann, welcher von der obigen Kamera zum Beispiel nicht erfasst werden kann, mit welcher das Instrument selbst oder die Geometrie oder Abmessung bestimmter Teilbereiche davon erfasst werden können, wobei die Kamera ebenfalls mit der Recheneinheit verbunden ist, so dass in der Recheneinheit ein Vergleich der von der Kamera optisch erfassten Instrumenten-Daten mit den in dem Speicher abgelegten Präkalibrier-Daten durchgeführt werden kann. Dabei ist die Kamera zur Erfassung der Marker-Positionen identisch mit der Kamera zur optischen Erfassung des Instrumentes. Ebenso ist es möglich mit der gleichen Kamera die Position der Marker zu erfassen und so das mit den Markern verbundene Instrument zu tracken und das Instrument selbst oder Abmessungen davon zu erfassen.

Weiterhin bezieht sich die Erfindung auf ein System mit einer wie oben beschriebenen Vorrichtung und einem Instrument, an welchem mindestens ein Marker angebracht ist.

Bei einem erfindungsgemäßen Verfahren zum Kalibrieren, Verifizieren oder Validieren eines bevorzugt medizinisch verwendbaren Instrumentes oder Implantats (nachfolgend auch als Instrument) bezeichnet, an welchem mindestens ein Marker und bevorzugt drei Marker zum Beispiel in Form eines so genannten Referenzsternes oder mehrere Marker mit bekannter fester oder sich zum Beispiel in Abhängigkeit von der Konfiguration des Instrumentes veränderbarer Geometrie angebracht sind, wird die Position des Instrumentes im Raum auf bekannte Art mittels eines Navigationssystems zum Beispiel mit einer IR-Stereokamera anhand der Marker, welche zum Beispiel als reflektierende Oberflächen oder Kugeln ausgebildet sein können, ermittelt. Zur Erfassung der Position des Instrumentes ist eine in mindestens eine IR-Kamera integrierte Kamera vorgesehen, welche zum Beispiel sichtbares Licht erfassen kann, welches von dem Instrument ausgesandt oder reflektiert wird, wobei die Kamera bevorzugt kalibriert ist und die Position der Kamera im Raum auch bekannt oder definiert ist. Weiterhin wird die Geometrie, also zum Beispiel eine oder mehrere Ansichten, Abbildungen oder Umrisse des Instrumentes aus einer oder verschiedenen Richtungen optisch mittels mindestens einer Kamera erfasst, wobei diese Kamera die gleiche ist, welche zum Erfassen der Position der Marker verwendet wird, oder auch eine von dieser Kamera verschiedene zweite Kamera sein kann, mit welcher Bilder zum Beispiel im sichtbaren Wellenlängenbereich aufgenommen werden können.

Die Geometrie oder Kalibrier-Daten des Instrumentes sind in einer Software oder einem Computer gespeichert, so dass die dreidimensionale Darstellung des Instrumentes zum Beispiel in einer Datenbank abgelegt ist. Diese gespeicherten so genannten Präkalibrierdaten werden mit der von der Kamera erfassten Geometrie des Instrumentes verglichen, um durch diesen Vergleich mit den optischen Erfassungsdaten festzustellen, ob die optisch erfassten und die tatsächliche Geometrie oder Ausbildung des Instrumentes darstellenden Daten mit den Präkalibrierdaten übereinstimmen. Es wird somit eine Zuordnung zwischen zum Beispiel im IR-Bereich erfassten sogenannten Tracking-Daten und einem im sichtbaren Bereich erfassten Kamerabild vorgenommen, wobei im Falle der Übereinstimmung der Kamerabilddaten mit den Präkalibrier-Daten das Instrument kalibriert bzw. verifiziert oder validiert ist und im Falle eines festgestellten Unterschiedes oder eine Abweichung von den Präkalibrier-Daten zum Beispiel eine Fehlermeldung ausgegeben werden kann, so dass das Instrument kalibriert werden muss oder die Präkalibrier-Daten, welche zur nachfolgenden Navigation verwendet werden, an die optischen Erfassungsdaten angepasst werden. Vorzugsweise werden aus einem dreidimensionalen Daten- oder Software-Modell des Instruments jeweils diejenigen Ansichten oder Umrisse des Instrumentenmodells berechnet, welche der mittels der Marker gemessenen Orientierung oder Relativ-Position des realen Instruments zur Kamera entsprechen.

Wenn man davon ausgeht, dass das vom Rechner berechnete Bild auf Basis der erfassten Position des als dreidimensionales Modell bekannten Instruments im Raum und des Wissens um die Kalibrierung der Video-Kamera die Darstellung oder Sicht oder ein Modell der Welt bzw. eines Instrumentes durch den Computer oder eine Software ist und die von einer Kamera erfassten Videoeingabedaten die Situation in der realen oder echten Welt, also zum Beispiel ein tatsächliches vorhandenes Instrument, zeigen, so würde im Falle einer genau kalibrierten Videokamera, also einer Kamera mit bekannter Position und Ausrichtung und Erfassungsbereich und eines registrierten oder kalibrierten medizinischen Instrumentes das vom Rechner berechnete Bild für ein präkalibriertes Instrument exakt mit dem Bild zusammenfallen, welches in der Videoeingabe gesehen wird. Falls dies nicht der Fall ist, ist entweder die Kalibrierung oder Abstimmung der Kamera fehlerhaft oder das Instrument entspricht nicht den Präkalibrier-Daten, da es zum Beispiel aufgrund häufigen Gebrauchs verbogen wurde. Wenn angenommen wird, dass die Kalibrierung der Kamera während des gesamten Verfahrens korrekt ist, so kann mit dem von der Kamera erfassbaren kalibrierten Volumen eine zuverlässige Verifikation von Instrumenten, zu welchen Präkalibrier-Daten vorliegen, durchgeführt werden.

Die zum Beispiel in einem Computer gespeicherten Daten, welche die Geometrie und optional auch mögliche Freiheitsgrade des Instrumentes definieren, sind als Präkalibrier-Daten zum Beispiel als eine Beschreibung des dreidimensionalen Objektes für ein Navigationssystem zum Beispiel in einer Datenbank gespeichert und können beispielsweise ein dreidimensionales Modell darstellen, welches die genaue Form eines Objekts oder Instruments und die Position eines jeden Markers oder Referenz-Arrays auf dem Objekt beschreibt. Das Navigationssystem oder ein Computer kann das dreidimensionale Modell darstellen, welches dem Instrument entspricht, das der Chirurg verwendet. Die Beschreibung des prakalibrierten Instrumentes kann zum Beispiel die Information umfassen, welche Bereiche oder funktionelle Stellen oder Flächen des Instrumentes verifiziert werden müssen. Ebenso können Informationen als Präkalibrier-Daten gespeichert werden, mit welchen mögliche Formen, welche das Instrument annehmen kann, festgelegt werden, wie zum Beispiel Informationen bezüglich möglicherweise vorhandener Gelenke und Bewegungsmöglichkeiten oder allgemein Informationen bezüglich der Veränderungsmöglichkeiten oder Freiheitsgrade des Instrumentes.

Ein kalibriertes Videosignal ist eine Eingabe, welche zum Beispiel von einer Standardvideokamera erhalten wird und dessen Eigenschaften oder Parameter, wie zum Beispiel die Position und/oder die Erfassungsfunktion der Kamera, bestimmt und für eine so genannte "virtuelle Kamera" berechnet werden. Diese virtuelle Kamera wird von dem Computer verwendet, um Bilder basierend auf dreidimensionalen Objekten zum Beispiel durch eine Projektion in Erfassungsrichtung der realen Kamera zu berechnen, welche mit den tatsächlich vorhandenen oder erfassten Ansichten oder Objekten übereinstimmen, so dass, wenn die Videokamera zum Beispiel auf ein würfelförmiges Objekt mit bekannten Abmessungen gerichtet ist, die Position des würfelförmigen Objektes im dreidimensionalen Raum nach dem Kalibrieren des Kameravolumens anhand der Bildinformationen bestimmt werden kann. Eine zusätzliche Information kann nun zum Beispiel in das von der Kamera aufgenommene Videobild eingeblendet werden, so dass diese zusätzliche Information, wie zum Beispiel eine virtuelle Darstellung des zu verifizierenden Instrumentes, wie ein Teil der von der Kamera aufgenommenen Szene wirkt.

Das kalibrierte Videosignal wird verwendet, um präkalibrierte Instrumente zu verifizieren und validieren, wobei kein anderes Objekt, wie zum Beispiel eine Anlagefläche, verwendet werden muss, so dass der Arbeitsbereich des Chirurgen nicht durch ein zusätzliches Objekt oder Instrument beeinträchtigt wird. Die erfindungsgemäß mögliche berührungslose Verifikation erfordert lediglich, dass ein Chirurg das Instrument, welches kalibriert werden soll, so hält, dass die Videokamera zumindest einen Teilumriss erfassen oder eine Teilansicht des Objektes von mindestens einer Seite aufnehmen kann, wobei eine nachgeschaltete Software, wie zum Beispiel das Navigationssystem, automatisch die Korrektheit der erfassten Form durch einen Vergleich mit den Präkalibrier-Daten bestimmen kann.

Falls ein Instrument mit einer komplexeren Form kalibriert werden soll, so kann es erforderlich sein, dass der Benutzer das Instrument bewegt und/oder dreht, um mehrere Ansichten des Objektes mit der Kamera aufzunehmen, wobei eine Software zum Beispiel eine entsprechende Instruktion zur Bewegung des Instrumentes ausgeben kann. Optional oder ergänzend können auch weitere Kameras zur Erfassung des Instruments aus unterschiedlichen Richtungen vorgesehen sein.

Durch das erfindungsgemäße Verfahren kann sichergestellt werden, dass nur kalibrierte Instrumente oder Implantate für chirurgische Verfahren verwendet werden, da zum Beispiel im Falle eines Abweichens der Form eines zu verwendenden Instruments von den Präkalibrier-Daten eine Fehlermeldung ausgegeben werden kann oder das Navigationssystem ein Navigieren des als fehlerhaft erkannten Instrumentes nicht ermöglicht.

Da es erfindungsgemäß nicht mehr erforderlich ist, ein Instrument zum Kalibrieren an eine Referenzfläche anzulegen, wird durch das erfindungsgemäße Verfahren auch die Handhabung von steril zu haltenden Instrumenten vereinfacht.

Vorzugsweise werden nicht nur eine, sondern mindestens zwei oder mehrere Seitenansichten des Instrumentes von einer optischen Kamera im sichtbaren Bereich des Lichts erfasst, wobei das Instrument auch im Sichtbereich der Kamera gedreht, verschoben oder rotiert werden kann. Vorzugsweise kann eine Sichtbarkeitsüberprüfung von bestimmten Punkten; wie zum Beispiel der Spitze eines Instrumentes, durchgeführt werden. Hierzu kann zum Beispiel überprüft werden, ob in den Präkalibrier-Daten definierte spezifische Punkte, wie zum Beispiel Eckpunkte, Kanten oder Spitzen des Instrumentes, auch in der optisch erfassten Aufnahme sichtbar sind oder verdeckt werden, wobei zum Beispiel im Falle der Verdeckung ein Signal ausgegeben werden kann, um einem Benutzer anzuzeigen, dass er das unverdeckte Instrument nochmals in die Sichtlinie der Kamera halten soll.

Es ist möglich, dass nur bestimmte Bereiche, wie zum Beispiel Eckpunkte, Kanten, eine Spitze oder funktionale Flächen des Instrumentes, welche charakteristisch oder für die Funktion des Instrumentes relevant sind, bezüglich einer Übereinstimmung mit den Präkalibrier-Daten geprüft werden, wobei eine diesbezügliche Information in einer Software und zum Beispiel in den Präkalibrier-Daten abgelegt sein kann.

Allgemein können die Präkalibrier-Daten Informationen zur Geometrie, Abmessung, räumlichen Anordnung von kombinierbaren Elementen, wie zum Beispiel eines Instruments mit austauschbaren Spitzen oder eines Instruments zur Platzierung von Implantaten in Verbindung mit dem gewählten Implantat und/oder zu möglichen Freiheitsgraden, wie zum Beispiel Gelenken oder Verformungsmöglichkeiten, des Instrumentes enthalten, so dass unter Verwendung der Präkalibrier-Daten zum Beispiel die Konfiguration oder der aktuelle Zustand eines zum Beispiel verstellbaren oder verformbaren Instrumentes erkannt werden kann, um diese Information über die tatsächliche Konfiguration des Instrumentes nachfolgend zum Beispiel im Rahmen der Behandlungsunterstützung oder für einen chirurgischen Eingriff mittels bildgeführter Chirurgie zu verwenden.

Ebenso ist es möglich, dass durch einen Vergleich der von einer Kamera erfassten Bilddaten mit den Präkalibrier-Daten überprüft wird, ob ein Instrument innerhalb einer vorgegebenen Spezifikation liegt, welche zum Beispiel in den Präkalibrier-Daten als Toleranz bezüglich Abmessungen des Instrumentes angegeben sein kann. Falls festgestellt wird, dass ein Instrument eine Toleranzgrenze überschreitet, kann zum Beispiel eine entsprechende Meldung ausgegeben werden.

Weiterhin ist es möglich, dass die von der Kamera aufgenommenen Daten bezüglich des tatsächlichen Zustandes oder der Konfiguration des Instrumentes verwendet werden, um die Präkalibrier-Daten anzupassen oder zu modifizieren, so dass die mittels der Kamera ermittelten Daten bezüglich der tatsächlichen Konfiguration eines Instrumentes zum Beispiel einem Navigationssystem zur Verfügung gestellt werden können, um dieses Instrument präzise zu navigieren.

Es ist möglich, dass ein den Präkalibrier-Daten entsprechendes Instrument und/oder ein tatsächlich von der Kamera erfasstes Instrument zum Beispiel auf einem Bildschirm angezeigt wird, wobei auch beide Instrumente, d.h. das reale und das virtuelle Instrument, gleichzeitig nebeneinander oder aufeinander abgebildet zum Beispiel als sogenanntes Überlappungsbild angezeigt werden. Dabei kann ein Vergleich charakteristischer Punkte, wie zum Beispiel Ecken und/oder Kanten, durchgeführt werden, um festzustellen, ob das reale Instrument mit dem virtuellen Instrument gemäß den Präkalibrier-Daten übereinstimmt oder davon abweicht.

Vorzugsweise wird die Kamera zur optischen Erfassung des Instrumentes im sichtbaren Bereich kalibriert. Hierzu kann beispielsweise ein optisches Muster, wie zum Beispiel ein Schachbrett oder ein Gegenstand mit bekannten Abmessungen vor eine Kamera gehalten werden, so dass anhand der von der Kamera erfassten Bilddaten zum Beispiel die Abmessungen eines im Sichtbereich der Kamera befindlichen Objektes optional unter Verwendung von Navigationsdaten ermittelt werden können.

Gemäß einem weiteren Aspekt bezieht sich die Erfindung auf ein Computerprogramm, welches, wenn es in einen Computer geladen ist oder auf einem Computer läuft, einen oder mehrere der oben beschriebenen Verfahrensschritte ausführt und zum Beispiel Programmabschnitte umfasst, mit welchen von einer optischen Kamera erfasste Bilddaten so ausgewertet werden können, dass Abmessungen oder die Geometrie eines sichtbaren Bereiches des Instrumentes optional unter Verwendung von Navigations-Daten bestimmt werden können, wobei die optisch erfassten Daten mit Präkalibrier-Daten verglichen werden können.

Weiterhin bezieht sich die Erfindung auf ein Programmspeichermedium oder ein Computerprogrammprodukt mit einem solchen Programm.

Die Erfindung wird nachfolgend anhand von Ausführungsbeispielen beschrieben werden. Es zeigen:
- Figur 1: eine erste Ausführungsform einer erfindungsgemäßen Vorrichtung;
- Figur 2: eine zweite Ausführungsform einer erfindungsgemäßen Vorrichtung;
- Figur 3: eine schematische Querschnittsansicht einer Kamera mit einem Prisma als Strahlleiter;
- Figur 4: eine schematische Querschnittsansicht einer Kamera des erfindungsgemäßen Kamerasystems gemäß einer zweiten Ausführungsform; und
- Figur 5: eine perspektivische Ansicht einer erfindungsgemäßen Stereoskopie-Kamera.

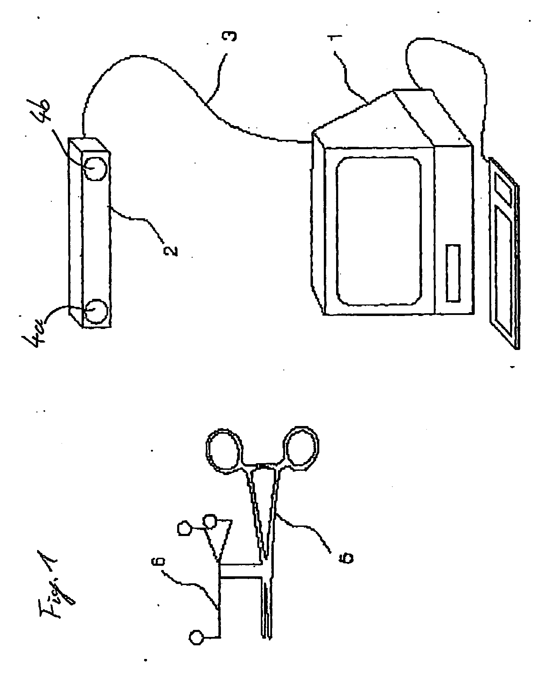

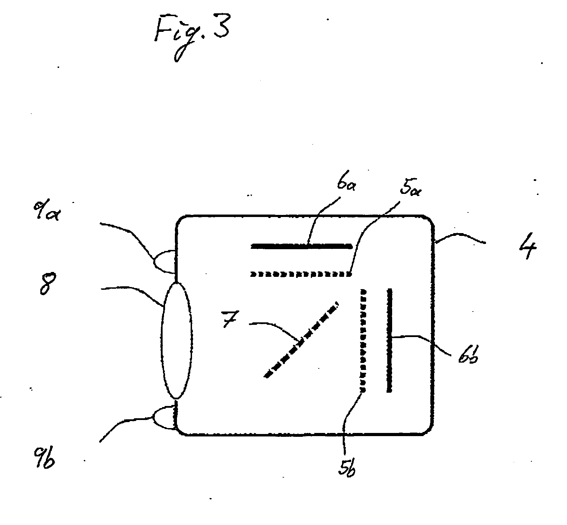

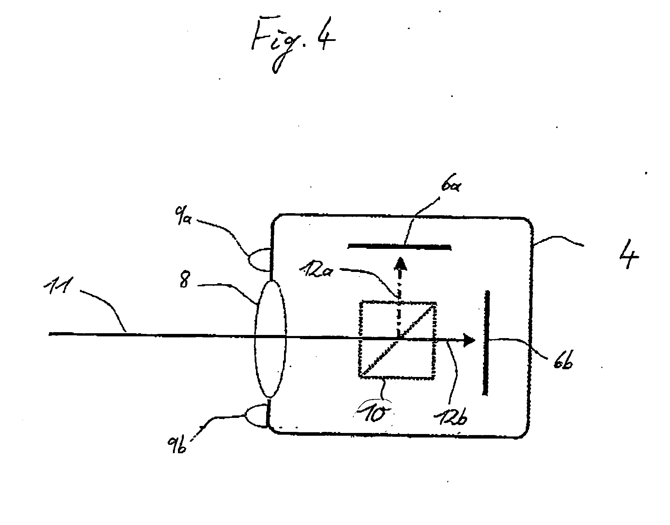

Figur 1 zeigt eine erste Ausführungsform eines erfindungsgemäßen Navigationssystems 1, welches mit einer optischen Kamera mit zwei Einzelkameras 4a und 4b gekoppelt ist. Das Navigationssystem 1 ist mittels einer Datenleitung, wie zum Beispiel einem Kabel 3 oder über Funk, mit einem optischen Tracking-System 2 bestehend aus zwei nachfolgend näher beschriebenen Kameras 4a, 4b verbunden, welche von den drei Markern des Referenzsternes 6 reflektierte IR-Lichtsignale erfassen können, um so die Position des mit dem Referenzstern 6 verbundenen medizinischen Instrumentes 5 in Gestalt einer Schere zu erfassen. Jede der Kameras 4a, 4b kann auch als Videokamera verwendet werden, wobei nicht TR, sondern sichtbares Licht erfasst wird. Die von dem optischen Tracking-System 2 im IR-Modus der Kameras 4a, 4b erfassten Daten des Referenzsternes 6 werden zusammen mit den im Videokamera-Modus erfassten Daten des Instrumentes 5 an die Recheneinheit 1 übersandt und zum Beispiel wie in der EP 1 667 067 A1 unter Bezugnahme auf deren Figuren 3 und 4 beschrieben ausgewertet.

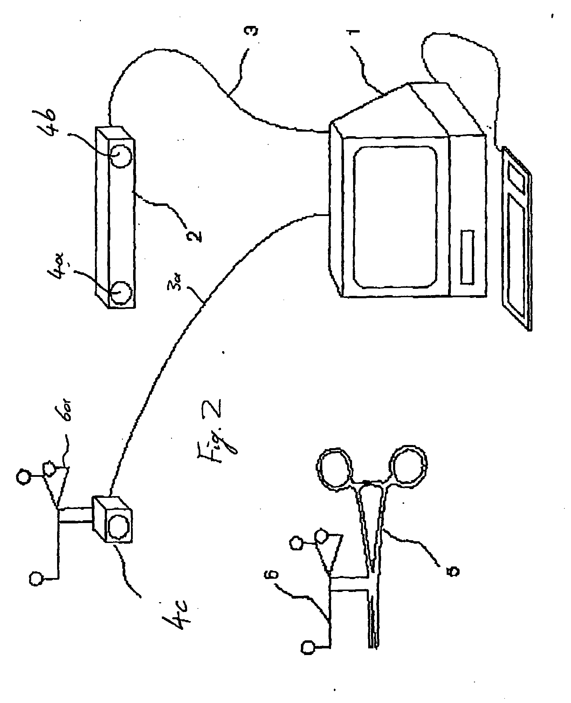

Durch die Integration der Videokamera-Funktion in das optische IR-Trackingsystem entfällt die Berechnung der aktuellen Position der Videokameras im Raum, das heißt ein zum Beispiel präkalibriertes Instrument 5 kann überprüft oder verifiziert werden, indem die von den Kameras 4a, 4b aufgenommenen Bilder im IR-Bereich zur Detektion der Marker und im sichtbaren Bereich zur Detektion der Form oder Geometrie des Instruments 5 in Relation zueinander gesetzt werden, um zum Beispiel eine Abweichung von einer vorgegebenen Instrumentenform festzustellen. Durch die Kalibrierung der Videokameras zum Beispiel bei der Herstellung des Systems erhält man die Informationen für die "virtuelle Kamera". Diese bleibt gültig, da die Position der Videokamera relativ zum Trackingsystem nach der Kalibrierung nicht mehr verändert wird. Bei einer losgelösten weiteren Videokamera 4c, wie sie in Figur 2 gezeigt wird, kann zum Beispiel zusätzlich die aktuelle Position der Kamera 4c bestimmt werden, um die Position der getrackten Instrumente 5 mit der "virtuellen Kamera" in Verbindung bringen zu können.

Figur 2 zeigt eine zweite Ausführungsform, wobei eine weitere Kamera 4c von dem optischen Tracking-System 2 mit den Kameras 4a und 4b losgelöst ist und über eine separate Datenverbindung, wie zum Beispiel ein Kabel 3a, mit der Recheneinheit 1 verbunden ist. Eine solche Anordnung ermöglicht es die Instrumentenerfassung durch die zusätzliche Kamera 4c flexibler zu gestalten, da diese unabhängig vom Tracking-System 2 positioniert werden kann, um zum Beispiel das Instrument 5 aus mehreren Richtungen zu erfassen. Hierzu muss ausgehend von dem Koordinatensystem der zusätzlichen Kamera 4c der Abstand des Instrumentes 5 von der Kamera 4c ermittelt werden, um die von der Kamera 4c erfassten Bilddaten so auswerten zu können, dass hieraus Abmessungen oder die Geometrie des Instrumentes 5 ermittelt werden kann. Da die Kamera 4c mit einem Referenzstern 6a verbunden ist, so dass der Referenzstern 6a zum Beispiel vom Tracking-System 2 erfasst werden kann und hieraus die Lage der zusätzlichen Kamera 4c im Raum und somit auch aus der von dem Tracking-System 2 erfassten Lage des Instrumentes 5 im Raum die Relativposition zwischen Kamera 4c und Instrument 5 berechnet werden kann, kann der Abstand des Instrumentes 5 von der Videokamera 4 bestimmt werden. Somit ist es zum Beispiel möglich ein Instrument mittels dreier Kameras 4a, 4b und 4c, welche jeweils IR und sichtbares Licht erfassen können, zu kalibrieren.

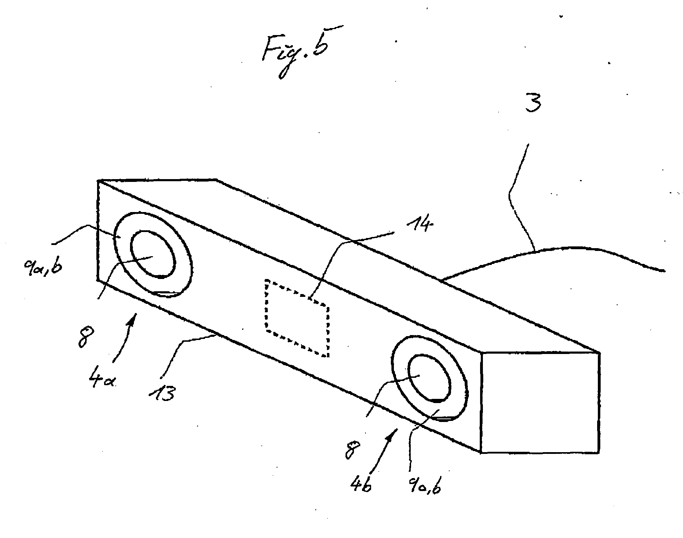

Figur 3 zeigt eine perspektivische Querschnittsansicht einer Kamera 4, wobei im Lichteintrittsbereich der Kamera 4 als Optik beispielhaft eine Linse 8 gezeigt ist, welche von Leuchtdioden 9a und 9b umgeben ist, die zum Beispiel ringförmig um die Linse 8 herum angeordnet sein können, wie in Figur 5 gezeigt. Ein durch die Optik oder Linse 8 eintretender Lichtstrahl trifft auf den halbdurchlässigen Spiegel 7, welcher einen Teil des Lichtstrahles hindurch lässt, um auf den direkt im Strahlengang liegenden Filter 5b und das dahinter liegende Erfassungselement oder CCD-Element 6b zu treffen, so dass mittels des CCD-Elementes 6b Licht in dem Wellenlängenbereich erfasst werden kann, welcher von dem Filter 5b durchgelassen wird. Ein Teil des durch die Linse 8 eintretenden Lichtes wird von dem halbdurchlässigen Spiegel 7 in Richtung auf den zweiten Filter 5a reflektiert, welcher Licht in einem anderen Wellenlängenbereich durchlässt als der Filter 5b, so dass dieses Licht des anderen Wellenlängenbereiches auf das in Richtung des durch den halbdurchlässigen Spiegel 7 reflektierten Lichtstrahles hinter dem Filter 5a liegende zweite CCD-Element 6a trifft, welches Licht in dem anderen Wellenlängenbereich erfassen kann. In Abhängigkeit von der Art der verwendeten Filter 5a, 5b kann der Wellenlängenbereich eingestellt werden, welcher von den CCD-Elementen 6a und/oder 6b nacheinander oder auch gleichzeitig erfasst wird.

Es wird angemerkt, dass in dem in Figur 3 gezeigten Ausführungsbeispiel auch auf einen der Filter 5a, 5b verzichtet werden kann, um zum Beispiel mittels des nicht durch einen Filter abgeschirmten CCD-Elementes sichtbares Licht zu erfassen, wobei beispielsweise ein Infrarot-Filter vor das andere CCD-Element gesetzt werden kann.

Mittels der Lichtquelle 9a kann zum Beispiel der Bereich vor der Kamera 4 mit Infrarot-Licht beleuchtet werden, um zum Beispiel die Detektion von reflektierenden Markern zu verbessern. Die zweite Lichtquelle 9b kann zum Beispiel Licht in einem anderen Wellenlängenbereich, wie zum Beispiel sichtbares Licht oder auch UV-Licht, aussenden, um zum Beispiel die Erfassung von Objekten durch Licht im sichtbaren Bereich zu verbessern. Jedoch kann je nach Anwendungsfall auch auf eine oder alle Beleuchtungselemente 9 verzichtet werden.

Es wird angemerkt, dass die Position der CCD-Elemente 6a und 6b in Bezug auf den halbdurchlässigen Spiegel 7 unterschiedlich sein können, das heißt die CCD-Elemente 6a und 6b können von dem Spiegel 7 unterschiedlich weit entfernt sein, da sich Licht verschiedener Wellenlängen nicht exakt auf den gleichen Punkt fokussiert. Es ist auch möglich das optische Element 8 variabel, zum Beispiel ähnlich wie bei einer an sich bekannten Foto- oder Video-kamera, auszugestalten, um je nach gewünschtem Licht-Erfassungsbereich das Licht auf den jeweiligen Sensor 6a oder 6b zu fokussieren.

Figur 4 zeigt eine Kamera 4, wobei an Stelle des in Figur 3 gezeigten halbdurchlässigen Spiegels 7 ein Strahlteilungsprisma 10 vorgesehen ist. Das auf das Prisma 10 eintreffende Licht 11 wird aufgeteilt in Licht eines ersten Wellenlängenbereiches 12b, welches durch das Prisma 10 hindurch tritt und auf das erste CCD-Element 6b trifft. Licht eines zweiten Wellenlängenbereiches kann durch das Prisma 10 gebrochen werden und wird als abgelenktes Licht 12a auf das zweite CCD-Element 6a gelenkt. Somit kann auch auf Filter verzichtet werden und eine gleichzeitige zum Beispiel kontinuierliche Erfassung eines Objektes durch die beiden CCD-Elemente 6a und 6b ist möglich.

Figur 5 zeigt ein Ausführungsbeispiel einer Stereoskopie-Kamera, wobei in einem Gehäuse 13 zwei entfernt voneinander liegende zum Beispiel wie oben beschriebene Einzelkameras 4a und 4b angeordnet sind, wobei in der perspektivischen Ansicht gemäß Figur 5 die jeweiligen Linsen 8 und die Beleuchtungselemente oder LEDs 9 erkennbar sind. In dem Gehäuse 13 ist eine CPU 14 angeordnet, welche mit jeder der Kameras 4a, 4b und insbesondere mit den jeweiligen CCD-Elementen 6a und 6b verbunden ist und über eine Verbindung 3 die von den Kameras 4a und 4b erfassten Stereoskopie-Bilder in zwei unterschiedlichen Wellenlängenbereichen an die in den Figuren 1 und 2 gezeigte Recheneinheit 1 übermittelt.

Mittels der Recheneinheit oder CPU 14 kann zum Beispiel die Aufnahme der Bilder durch die Kameras 4a, 4b, die Einstellung der Optik 8 oder die Einstellung oder Positionierung eines Filters 5a, 5b, wie zum Beispiel Verschieben eines Filters vor einem oder mehreren der CCD-Elemente 6a, 6b, um Licht eines anderen Wellenlängenbereiches zu erfassen, gesteuert werden. Eine Synchronisation zwischen den CCD-Elementen 6a und 6b einer Kamera und den den jeweiligen CCD-Elementen 6a, 6b jeweils zugeordneten Beleuchtungselementen 9a, 9b kann ebenfalls mittels der CPU 14 erfolgen. Das Auswertungsergebnis oder auch die zum Beispiel nicht unmittelbar von der CPU 14 ausgewerteten optischen Signale können über die Verbindung 3 an ein anderes System übertragen werden.

## Patentansprüche

1. Stereoskopie-Kamerasystem mit mindestens zwei Kameraeinheiten (4a, 4b), wobei
- jede Kameraeinheit (4a, 4b) mindestens zwei Erfassungselemente (6a, 6b) aufweist, um ein optisches Signal zu erfassen,
**dadurch gekennzeichnet, dass**
- in mindestens einer oder jeder Kameraeinheit (4a, 4b) mindestens ein optischer Filter (5a, 5b) vorgesehen ist, welcher vor mindestens einem Erfassungselement (6a, 6b) angeordnet ist und die Erfassung von Licht in mindestens zwei verschiedenen Spektralbereichen ermöglicht, und ein halbdurchlässiger Spiegel (7) vorgesehen ist, um unterschiedliche Spektren oder Wellenlängen des in die Kamera eintretenden Lichtes auf die mindestens zwei Erfassungselemente (6a, 6b) zu lenken.

2. Kamerasystem nach dem vorhergehenden Anspruch, wobei ein Prisma (10) verwendet wird, um unterschiedliche Spektren oder Wellenlängen des in die Kamera eintretenden Lichtes auf die mindestens zwei Erfassungselemente (6a, 6b) zu lenken.

3. Kamerasystem nach einem der vorhergehenden Ansprüche, wobei das Erfassungselement ein elektronisches Erfassungselement, ein CCD-Chip, ein CMOS Sensor, ein Zeilensensor, oder ein Sensor-Array ist.

4. Kamerasystem nach einem der vorhergehenden Ansprüche, wobei das Element ein bewegbarer Spiegel (7) ist.

5. Kamerasystem nach einem der vorhergehenden Ansprüche, wobei mindestens ein optischer Filter (5a, 5b) innerhalb der Kamera (4) bewegbar oder in seinen optischen Eigenschaften veränderbar ist, um je nach Position oder Einstellung unterschiedliche Wellenlängenbereiche des Lichtes zu filtern.

6. Kamerasystem nach einem der vorhergehenden Ansprüche, wobei ein optisches System oder eine Linse (8) vorgesehen ist, durch welches Licht in die Kamera (4) eintreten kann.

7. Kamerasystem nach dem vorhergehenden Anspruch, wobei das optische System (8) ein einstellbares oder veränderbares optisches System ist.

8. Navigationssystem mit einem Kamerasystem nach einem der vorhergehenden Ansprüche.

9. Verfahren zum Kalibrieren eines Instruments (5) oder Implantats, an welchem mindestens ein Marker (6) angebracht ist, wobei
- die Position des Instrumentes (5) oder Implantats im Raum mittels des mindestens einen Markers (6) durch Erfassung von Licht in einem ersten Wellenlängenbereich mit einem Stereoskopie-Kamerasystem mit mindestens zwei Kameraeinheiten (4a, 4b) ermittelt wird,
- die Umrisse oder Ansicht oder Geometrie des Instrumentes (5) oder Implantats von mindestens einer Seite optisch in einem anderen zweiten Wellenlängenbereich mit dem gleichen Kamerasystem (4a, 4b) erfasst wird, wobei in mindestens einer oder jeder Kameraeinheit (4a, 4b) ein halbdurchlässiger Spiegel (7) und mindestens ein optischer Filter (5a, 5b) vorgesehen sind, um unterschiedliche Spektren oder Wellenlängen des in die Kamera eintretenden Lichtes auf mehrere jeweils verschiedene Erfassungselemente (6a, 6b) zu lenken, wobei der mindestens eine optische Filter (5a, 5b) vor mindestens einem Erfassungselement (6a, 6b) angeordnet ist, und
- von einer mit dem Stereoskopiekamerasystem verbundenen Recheneinheit mit Speicher mit korrespondierenden Umrissen, Ansichten oder Geometrien von in dem Speicher gespeicherten Präkalibrier-Daten des Instrumentes (5) oder Implantats zur Darstellung eines Instrumenten-Modells verglichen wird, um zu ermitteln, ob das Instrument (5) oder Implantat kalibriert ist.

10. Verfahren nach dem vorhergehenden Anspruch, wobei aus den optisch in zwei verschiedenen Wellenlängenbereichen erfassten Marker- und Instrumentendaten nach einem Vergleich mit den Präkalibrier-Daten die aktuelle Konfiguration des Instruments (5) ermittelt wird.

11. Computerprogramm, welches, das Verfahren nach einem der zwei vorhergehenden Ansprüche ausführt oder steuert wenn es in die Recheneinheit geladen wird oder auf der Recheneinheit läuft.

12. Programmspeichermedium mit dem Programm nach dem vorhergehenden Anspruch.

## Claims

1. A stereoscopic camera system comprising at least two camera units (4a, 4b), wherein
- each camera unit (4a, 4b) comprises at least two detection elements (6a, 6b) for detecting an optical signal,
**characterised in that**
- in at least one or each camera unit (4a, 4b), at least one optical filter (5a, 5b) is provided which is arranged in front of at least one detection element (6a, 6b) and enables light to be detected in at least two different spectral ranges, and a semi-transparent mirror (7) is provided in order to deflect, onto the at least two detection elements (6a, 6b), different spectra or wavelengths of the light entering the camera.

2. The camera system according to the preceding claim, wherein a prism (10) is used to deflect, onto the at least two detection elements (6a, 6b), different spectra or wavelengths of the light entering the camera.

3. The camera system according to any one of the preceding claims, wherein the detection element is an electronic detection element, a CCD chip, a CMOS sensor, a line sensor or a sensor array.

4. The camera system according to any one of the preceding claims, wherein the element is a movable mirror (7).

5. The camera system according to any one of the preceding claims, wherein at least one optical filter (5a, 5b) can be moved within the camera (4) or changed in its optical properties in order to filter different wavelength ranges of the light depending on its position or setting.

6. The camera system according to any one of the preceding claims, wherein an optical system or a lens (8) is provided, through which light can enter the camera (4).

7. The camera system according to the preceding claim, wherein the optical system (8) is a settable or variable optical system.

8. A navigation system comprising a camera system according to any one of the preceding claims.

9. A method for calibrating an instrument (5) or implant to which at least one marker (6) is attached, wherein:
- the spatial position of the instrument (5) or implant is ascertained by means of the at least one marker (6) by detecting light in a first wavelength range using a stereoscopic camera system comprising at least two camera units (4a, 4b);
- the outlines or view or geometry of the instrument (5) or implant are optically detected from at least one side in another, second wavelength range using the same camera system (4a, 4b), wherein in at least one or each camera unit (4a, 4b), a semi-transparent mirror (7) and at least one optical filter (5a, 5b) are provided in order to deflect, onto a number of respectively different detection elements (6a, 6b), different spectra or wavelengths of the light entering the camera, wherein the at least one optical filter (5a, 5b) is arranged in front of at least one detection element (6a, 6b); and
- compared, by a computational unit which is connected to the stereoscopic camera system and comprises a memory, with corresponding outlines, views or geometries of pre-calibration data of the instrument (5) or implant for displaying an instrument model, which are stored in the memory, in order to ascertain whether the instrument (5) or implant is calibrated.

10. The method according to the preceding claim, wherein after a comparison with the pre-calibration data, the current configuration of the instrument (5) is ascertained from the marker and instrument data optically detected in two different wavelength ranges.

11. A computer program which performs or controls the method according to any one of the preceding two claims when it is loaded onto the computational unit or is running on the computational unit.

12. A program storage medium comprising the program according to the preceding claim.

## Revendications

1. Système de caméra stéréoscopique comportant au moins deux unités de caméra (4a, 4b), dans lequel
- chaque unité de caméra (4a, 4b) comporte au moins deux éléments de détection (6a, 6b) pour détecter un signal optique,
**caractérisé en ce que**
- au moins un filtre optique (5a, 5b) est prévu dans au moins une ou chaque unité de caméra (4a, 4b), le dit filtre étant disposé devant au moins un élément de détection (6a, 6b) et permettant la détection de lumière dans au moins deux différents domaines spectraux, et un miroir semi-transparent (7) est prévu pour diriger différents spectres ou différentes longueurs d'onde de la lumière pénétrant dans la caméra sur les au moins deux éléments de détection (6a, 6b).

2. Système de caméra selon la revendication précédente, dans lequel un prisme (10) est utilisé pour diriger différents spectres ou différentes longueurs d'onde de la lumière pénétrant dans la caméra sur les au moins deux éléments de détection (6a, 6b).

3. Système de caméra selon l'une quelconque des revendications précédentes, dans lequel l'élément de détection est un élément de détection électronique, une puce CCD, un capteur CMOS, un capteur de ligne ou une mosaïque de capteurs.

4. Système de caméra selon l'une quelconque des revendications précédentes, dans lequel l'élément est un miroir mobile (7).

5. Système de caméra selon l'une quelconque des revendications précédentes, dans lequel au moins un filtre optique (5a, 5b) est mobile à l'intérieur de la caméra (4) ou dont les propriétés optiques peuvent être changées afin de filtrer différentes plages de longueurs d'onde de la lumière en fonction de la position ou du réglage.

6. Système de caméra selon l'une quelconque des revendications précédentes, dans lequel un système optique ou une lentille (8) est prévu, à travers lequel la lumière peut pénétrer dans la caméra (4).

7. Système de caméra selon la revendication précédente, dans lequel le système optique (8) est un système optique réglable ou changeable.

8. Système de navigation comportant un système de caméra selon l'une quelconque des revendications précédentes.

9. Procédé pour calibrer un instrument (5) ou un implant, sur lequel est fixé au moins un marqueur (6), comportant les étapes consistant à :
- déterminer la position de l'instrument (5) ou de l'implant dans l'espace au moyen du au moins un marqueur (6) en détectant la lumière dans une première plage de longueurs d'onde à l'aide d'un système de caméra stéréoscopique comportant au moins deux unités de caméra (4a, 4b),
- détecter optiquement les contours ou la vue ou la géométrie de l'instrument (5) ou de l'implant à partir d'au moins un côté dans une seconde autre plage de longueurs d'onde à l'aide du même système de caméra (4a, 4b), un miroir semi-transparent (7) et au moins un filtre optique (5a, 5b) étant prévus dans au moins une ou chaque unité de caméra (4a, 4b) pour diriger différents spectres ou différentes longueurs d'onde de la lumière pénétrant dans la caméra sur plusieurs différents éléments de détection respectifs (6a, 6b), le au moins un filtre optique (5a, 5b) étant disposé devant au moins un élément de détection (6a, 6b), et
- à partir d'une unité de calcul reliée au système de caméra stéréoscopique et comportant une mémoire, établir une comparaison avec des contours, des vues ou des géométries correspondantes à partir de données de pré-calibrage de l'instrument (5) ou de l'implant, mémorisées dans la mémoire, pour afficher un modèle d'instrument afin de déterminer si l'instrument (5) ou l'implant est calibré.

10. Procédé selon la revendication précédente, dans lequel à partir des données de marqueur et d'instrument détectées optiquement dans deux plages de longueurs d'onde différentes, la configuration actuelle de l'instrument (5) est déterminée après comparaison avec les données de pré-calibrage.

11. Programme informatique, qui met en oeuvre ou commande le procédé selon l'une quelconque des deux revendications précédentes lorsqu'il s'exécute dans l'unité de calcul ou sur l'unité de calcul.

12. Support de mémorisation de programme comportant le programme selon la revendication précédente.
